# EUROPÄISCHE PATENTANMELDUNG

(11) **EP 0 613 762 A1**
(43) Veröffentlichungstag der Anmeldung: **07.09.1994**
(21) Anmeldenummer: 94200424.3
(22) Anmeldetag: 18.02.1994
(51) Int. Cl.: B25J 3/00, B25J 9/10, B25J 13/02, B25J 15/00, A61B 19/00

(54) **Chirurgischer Manipulator**

(30) Priorität: 04.03.1993 DE 4306786; 07.08.1993 DE 4326618
(71) Anmelder: DAUM GmbH, D-19061 Schwerin (DE)
(72) Erfinder: Daum, Wolfgang, D-19067 Neu Schlagsdorf (DE); Schmiedeke, Jörg, D-19053 Schwerin (DE)

(57) **Zusammenfassung**

Ein Manipulator, in dessen Halterung (1) der proximalen Seite die Hand (3-8) und der Arm (2) des Operateurs eingelegt wird. Hier wird die Bewegung der menschlichen Extremität aufgenommen. Mittels Drähten (22) wird die Bewegung an die distale Extremität weitergegeben, die nach Form und Funktion der menschlichen entspricht. Der Manipulator kann z.B. durch einen in der Chirurgie üblichen Trokar (24) geschoben werden und dient dann zum Hantieren im Operationsgebiet.

## Beschreibung

Die Erfindung betrifft einen mechanischen Manipulator zum Arbeiten in unzugänglichen Gebieten und Hohlräumen nach dem Oberbegriff des Anspruchs 1.

In der neueren endoskopisch arbeitenden Minimal Invasiven Chirurgie, aber auch in vielen Bereichen der Technik ist es oft schwierig, in unzugänglichen Bereichen handgerecht zu arbeiten. Es sind daher viele Apparaturen entwickelt worden, um spezielle Greif- und Hantierarbeiten auszuführen. Der Nachteil dieser Spezialwerkzeuge ist jedoch, daß diese Geräte immer wieder beim Voranschreiten der Operation ausgewechselt werden müssen. Es wäre daher von Vorteil, in unzugänglichen Bereichen mit einer verkleinerten künstlichen Hand arbeiten zu können.

Die Erfindung widmet sich dem Problem, dem Operateur einen Manipulator zu schaffen, der
· die Bewegungen des menschlichen Armes mit Hand und Fingern direkt aufnimmt und diese direkt an
· eine der Form und Funktion nach gleiche Einheit weitergibt, die
· sich durch enge Kanäle, wie den Trokaren der heutigen Minimal Invasiven Chirurgie, in das Operationsgebiet schieben läßt, dort handgleich aggiert und dabei
· einfach aufgebaut, so daß er leicht tragbar ist.

Rein mechanisch arbeitende Vorrichtungen dieser Art sind bisher nicht bekannt. Vorrichtungen zum Aufnehmen der Bewegung menschlicher Bewegungen zur Fernsteuerung von Greifeinrichtungen von Robotern sind bereits bekannt geworden.
PS-US-4,302,138 sowie PS-F-78 02714 zeigen eine Vorrichtung, die die Bewegung der menschlichen Hand entweder direkt durch an der Hand sitzende oder von der Hand über mechanische Gestänge an weiter entfernt befindlichen elektrischen Sensoren mißt. Diese geben das elektrische Signal zur Steuerung eines Greifers weiter.

Vorrichtungen, die der Form und Funktion nach wie menschliche Hände aggieren, sind bereits bekannt geworden, jedoch werden sie alle nicht direkt, rein mechanisch von den menschlichen Gliedern gesteuert. US-PS-4,834,761 zeigt hierzu eine Vorrichtung, die mittels eines Systems hydraulisch arbeitender Kolben betrieben und deren Bewegung mittels Bändern (Sehnen) über Flaschenzugkonstruktionen auf die Finger übertragen wird. US-PS-5,092,646 zeigt eine von Stellmotoren betriebene Vorrichtung, in der jeweils ein ganzer Finger einer Hand mittels dehnbaren Bändern über eine Doppelwindenkonstruktion gebeugt und über eine Spiralfeder gestreckt wird. Ebenfalls wird in PS-US-4,986,723 die Streckbewegung mit Hilfe einer Spiralfeder und die Beugbewegung mit Hilfe eines Flaschenzuges vollzogen. PS-US-4,921,293 zeigt ebenfalls mit Flaschenzugkonstruktionen bewegte Finger.

Die Übermittlung der Steuersignale des Bedienfeldes an die Greifervorrichtung erfolgt bei den benannten Apparaten elektrisch. Eine mechanisch arbeitende Übertragungen ist in DE-AS-2048563 bekannt geworden. Hier wird über Flaschenzüge ein fest installierter Arbeitsarm bewegt. Die vom Gerätearm ausgeführte Bewegung wird dabei jedoch nicht vom Arm der Bedienungsperson aufgenommen und übertragen, sondern von einem von dieser Person zu bewegenden Steuerarm. Dabei muß sich der Bediener laufend überlegen, wie er den Steuerarm zu lenken hat, um den Arbeitsarm in die gewünschte Lage zu bringen.

Das gleiche tritt in der in DE-PS-2939452 dargelegten Idee auf. Auch hier handelt es sich nicht um eine tragbare, sondern eine feststehende Apparatur. Beide Arme sind direkt drehbar über eine Achse miteinander verbunden, so daß das Schwenken des Steuerarmes zu einem entsprechenden Schwenken des Folgearmes führt. Aufgrund dieser Konstruktionsweise läßt sich jedoch die Vorrichtung nicht für Bewegungssteuerungen durch einen dünnen Kanal, wie dem eines Trokars, verwenden. Dies ist aber Ziel des Anspruchs 1 der hier vorgestellten Erfindung. Die zwei in der Schrift DE-PS-2939452 gezeigten Parallelogramme sind unmittelbarer Bestandteil der Arme, ohne die keine einzige Bewegung ausführbar ist. Die Bewegungsübertragung des Anspruchs 1 wird jedoch durch den Schub oder Zug an Drähten bzw. Fäden erreicht. Die Hebel bzw. pantographischen Einrichtungen des Anspruchs 2 dienen lediglich der Wegkorrektur dieser Drähte bzw. Fäden und sind nicht unmittelbarer Teil der Glieder. Die Bewegung der Vorrichtungsglieder ist auch ohne diese Einrichtungen möglich, so daß über diese lediglich eine Anpassung der Bowdenzugbewegung geschieht. Diese Anpassung ist z.B. dann wichtig, wenn an eine proximal vorhandene Hand eine neue kleinere distale gesetzt werden soll. Spezialbewegungen können in der weiter unten angegebenen Weise hierdurch erreicht werden.

All diese Vorrichtungen sind für den Einsatz in unzugänglichen Bereichen kleiner Dimensionen - wie z.B. der Bauchhöhle des Menschen - aufgrund ihrer Ausmaße und ihres komplizierten Aufbaus ungeeignet.

Bei der in Anspruch 1 vorgestellten mechanischen Manipulatorvorrichtungen ist es aufgrund der geringen Abmessungen oft schwierig, kostengünstige mechanische Konstruktionen für die distalen Manipulatorteile zu schaffen. Insbesondere ist es bei besonders kleinen distalen Händen schwierig, die Gelenke zu gestalten. Weiterhin erweisen sich Konstruktionen mit Hohl- und Zwischenräumen, wie sie bei herkömmlichen Gelenken zu finden sind, als schwierig zu reinigen. Dies ist besonders in der Chirurgie von Bedeutung.

Der Erfindung liegt daher weiterhin die Aufgabe zugrunde, eine leicht zu miniaturisierende distale Hand-Arm-Vorrichtung zu schaffen, die auch einfach zu reinigen ist.

Anhand folgender Figuren soll die hier vorgelegte Erfindung erläutert werden:
- Fig. 1: Vorrichtung in Gesamtansicht,
- Fig. 2: Prinzip der Bewegungsübertragung anhand eines Fingers mit Ausschnittsbildern zur Verdeutlichung der Winkelbezeichnungen sowie
- Fig. 3: Vorzugsbauform der Halterung als Handschuh mit eingelassenen Bowdenzügen,
- Fig. 4: Ansatz zweier Fäden an einem distalen Finger,
- Fig. 5: Bowdenzugansatz zur Aufnahme der Drehbewegung der Hand um die Längsachse des Unterarmes,
- Fig. 6: a - Verkleinerung der menschlichen Handbewegung mittels einer pantographischen Einrichtung,
b - Erzeugung und Bewegungsablauf einer nichtlinearen Übersetzung und
c - Beispiel einer menschenhandfremden Spezialanwendung,
- Fig. 7: Gelenk einer besonders kleinen distalen Vorrichtungsextremität,
- Fig. 8: Ansatz der Bowdenzüge an der Fingeroberseite,
- Fig. 9a: Längsschnitt eines ausgestreckten distalen Fingers,
- Fig. 9b: Längsschnitt eines gekrümmten distalen Fingers,
- Fig. 10: Querschnitt durch einen Finger mit dünnem Blech und
- Fig. 11: Längsschnitt eines distalen Fingers einer in Gummi eingegossenen Manipulatorhand,
- Fig. 12: Seitenansicht und Draufsicht eines Manipulators mit Koppelglied auf der Hand,
- Fig. 13: Seitenansicht eines Manipulators mit Koppelglied im Handinneren und
- Fig. 14: schematische Schnittzeichnung einer Schutzvorrichtung des distalen Manipulatorteils.

Der Anspruch 1 soll in den ersten vier Figuren erläutert werden.

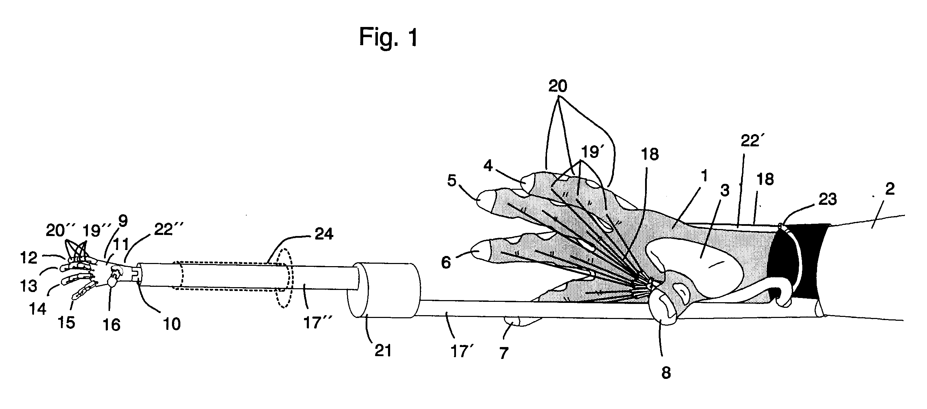

In Fig. 1 ist eine Gesamtdarstellung einer möglichen Form der Vorrichtung gezeigt. Sie besteht aus der Halterung 1 für die menschliche Extremität Unterarm 2, Handkörper 3 und den fünf Fingern 4, 5, 6, 7 und 8 mit Einzelgliedern. Die Halterung 1 ist hier als eine Art Gummihandschuh ausgeführt, die paßgenau die menschliche Extremität aufnimmt und deren Bewegung leicht und ohne zu behindern mitmacht. Weiterhin besteht die Apparatur aus der distal gelegenen Extremität 9, die aus entsprechenden Gliedern, nämlich dem Unterarm 10, Handkörper 11 und den fünf Fingern 12, 13, 14, 15 und 16 aufgebaut ist. Diese entsprechen ihrer Form und Funktion nach der menschlichen Extremität. In einem starren Kanal 17' und 17'' liegen die Drähte 18, die mit ihren proximalen Enden 19' beweglich an den Gliedern 20 der gummiartigen Halterung 1 und mit ihren distalen Enden 19'' beweglich an den Gliedern 20'' der distalen Extremität befestigt sind. Über ein weiter unten beschriebenes Koppelglied 21 wird die Richtung und Weglänge der durch die menschliche Hand bewegten Drähte 18 an die Erfordernisse der distalen Extremität angepaßt.

Da die Glieder der proximalen Extremität direkt und mechanisch über ein Koppelglied miteinander verbunden sind, kann durch Bewegen eines Gliedes der menschlichen Extremität das entsprechende distale Glied bewegt werden. Wird in Fig.1 z.B. der Handkörper 3 gegen den Unterarm 2 in Richtung Rückhand hochgebeugt, so wird der Draht 22 in seine Hülle 23 geschoben - Bowdenzug -. Diese Bewegung wird im Koppelglied 21 auf die Gegebenheiten der distalen Hand angepaßt, so daß das distale Ende 22'' den distalen Handkörper 11 durch Ziehen an diesem gegen den distalen Unterarm 10 beugt.

Die distale Hand kann durch diesen Bewegungsmechanismus so zusammengelegt werden, daß diese mit dem vorderen Teil des Kanals 17'' durch einen Trokar 24 an bzw. in das Operationsgebiet geschoben werden kann.

Die Bewegung des Oberarms gegen den Unterarm ist in diesem Beispiel nicht gezeigt. Die Bowdenzüge können verschiedene Dimensionen aufweisen. So kann es ratsam sein, dickere Züge für die Übertragung der Bewegung größerer menschlicher Gliedmaßen und dünnere für die Übertragung kleinerer zu benutzen. Ebenso können verschiedene Materialien wie Metalle oder Kunststoffe genutzt werden. Es kann z.B. sinnvoll sein, Kunststoffe wie PTFE (Teflon®) oder Werkstoffe mit ähnlich geringen Reibungskoeffizienten zu nutzen, um das Gleiten der Züge in den Hüllen zu optimieren.

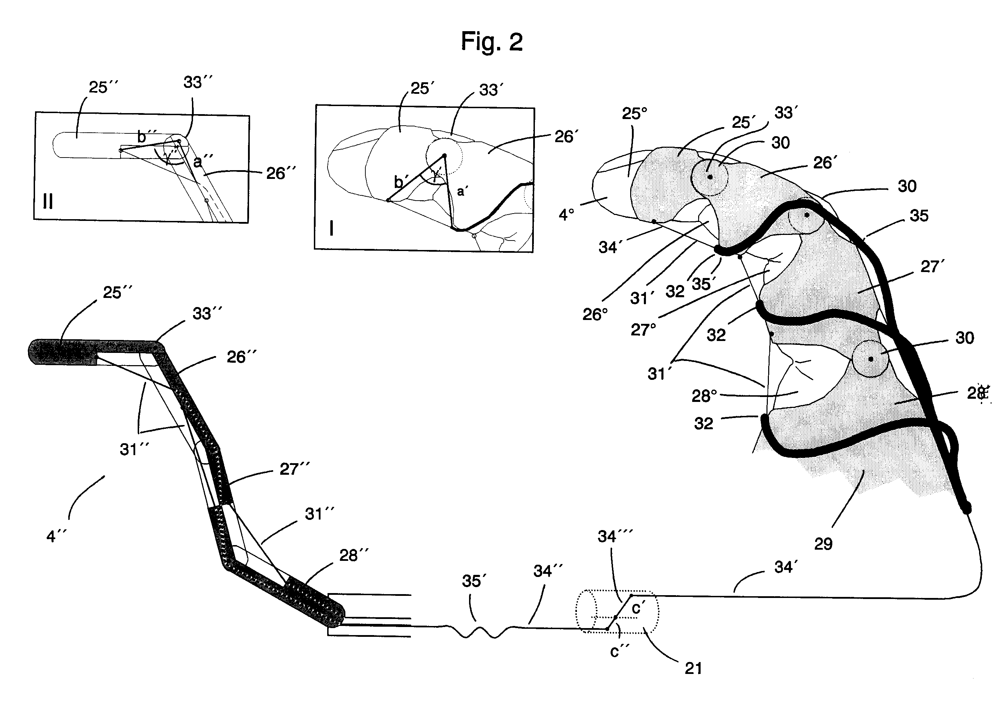

In Figur 2 soll der Übertragungsmechanismus näher erläutert werden. Dargestellt ist ein menschlicher Finger 4° mit dem Fingerendglied 250, dem Fingermittenglied 26°, Fingergrundglied 27° und dem entsprechenden Mittelhandteil 280. Der Finger paßt in die hier nicht als Gummihandschuh ausgeführte Halterung 29, die aus entsprechenden Gliedern 25', 26', 27' und 28' sowie aus Gelenken 30 besteht. Am distalen Ende befindet sich der zur besseren Darstellung zu groß gezeichnete Vorrichtungsfinger 4'' mit entsprechenden Gliedern 25'', 26'', 27'' und 28''. Anders als bei der Bewegungsübertragung in Fig. 1 laufen die proximalen Endteile 31' der Drähte hier nicht frei über die Hohlhand, sondern direkt in die am Nachbarglied befestigte Hülle 32, mit der sie eine Art Bowdenzug bilden. Am distalen Finger verlaufen die distalen Teile 31'' der Drähte teils in den Fingergliedern. Ebenfalls anders als in Fig. 1 ist der Kanal als nicht starr angenommen, was durch den kurvenartigen Verlauf 35'' des Drahtes im distalen Teil 34'' angedeutet ist. Aus Gründen der besseren Übersicht wird nur ein Draht gezeigt.

Beugt nun der Operateur sein Fingerendglied 25° gegen sein Fingermittenglied 26°, so beugen sich auch die entsprechenden Glieder 25' und 26' um deren gemeinsames Gelenk 33', da die Halterung die Fingerbewegung ohne Behinderung mitmacht. Der proximale Übertragungsdraht 34' wird dadurch in eine Bowdenzughülle 35' geschoben. In dem Koppelglied 21 wird durch einen Hebel 34''' die Bewegungsrichtung des Drahtes umgedreht. Dadurch kann nun am distalen Finger das Fingerendglied 25'' gegen das Fingermittenglied 26'' um das gemeinsame Gelenk 33'' gebeugt werden.

Der vom proximalen Teil 34' des Drahtes zurückgelegte Weg läßt sich über den Cosinussatz mit den im Teilbild I der Fig. 1 gezeigten Größen bestimmen. Diese ergeben sich aus dem Abstand b' der Drahtbefestigung zum Drehpunkt 33', dem Abstand a' der am Nachbarfingerglied befestigten Hüllenöffnung zum Drehpunkt und dem Beugungswinkel γ'. Entsprechende Größen a'', b'' und γ'' sind im Teilbild II für die distale Seite gezeigt. Über die Abstände a'', b'', a' und b' sowie die der Koppelstange c'' und c' läßt sich das Winkelübertragungsverhältnis γ''/γ' einstellen, welches für die meisten Anwendungen gleich 1 sein wird.

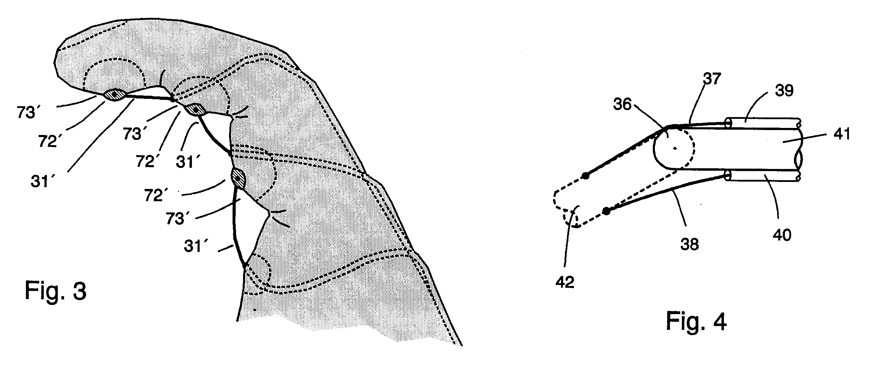

Zur Verdeutlichung der Winkelbeziehungen wurde in Fig. 2 eine Halterung mit Gelenken und daher eindeutigen Drehachsen gewählt. Die in Fig. 3 gezeigte Vorzugsbauform stellt jedoch den nur anhand eines Fingers dargestellten Handschuh dar, in dessen Gummimaterial die Bowdenzüge eingelassen sind. Die Drähte 31' sind dann beweglich z.B. an Achsen 72 oder mit hier nicht gezeigten Kugelgelenken an fest in den Handschuh eingelassenen Gelenkhalterungen 73' befestigt. Der Vorteil dieser Art Halterung ist die paßgenaue Form für die menschliche Extremität sowie die leichtgängige Bewegbarkeit.

Es muß darauf hingewiesen werden, daß der Aufbau des distalen Fingers aufgrund dessen Kleinheit durch einfach zu erstellende Teile erreicht wird. Weitere Bowdenzüge können für das seitliche Spreizen der Finger untereinander benutzt werden.

Anhand des Gelenkes 36 eines distalen Fingers soll in Fig. 4 gezeigt werden, daß statt der Drähte auch zwei Fäden 37 und 38 genutzt werden können. Die zu den Fäden gehörenden Hüllen 39 und 40 sind ober- und unterhalb eines Fingergliedes 41 angebracht. Durch das Lockern des Fadens 37 und Anziehen des Fadens 38 wird das Fingerglied 42 um das Gelenk 36 gebeugt und umgekehrt. Entsprechende Teile zur Fadenzugaufnahme können sich dann auch an den entsprechenden proximalen Gliedern der Vorrichtung befinden.

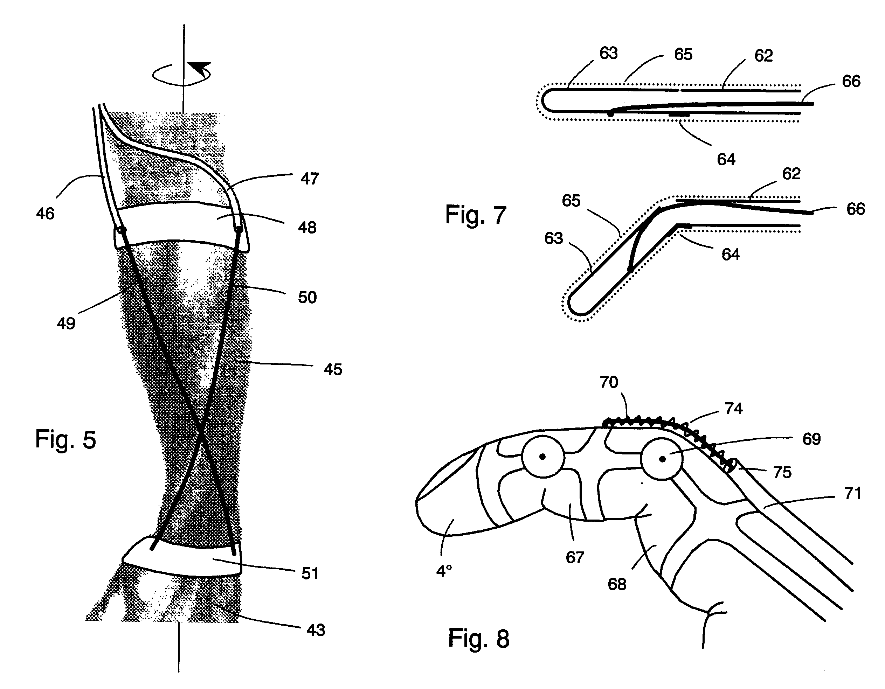

Fig. 5 zeigt die Aufnahme der Drehbewegung der menschlichen Hand 43 um die Achse 44 des Unterarmes 45 mittels zweier Bowdenzüge. Dabei enden die Hüllen 46 und 47 an dem Armteil 48 der Halterung und die sich auf dem Unterarm 45 kreuzenden Züge 49 und 50 am Handteil der Halterung. Wird nun die Hand 43, vom Unterarm 45 aus gesehen, entgegen dem Uhrzeigersinn in gezeigter Pfeilrichtung gedreht, so wird der Zug 50 in seine Hülle 47 geschoben und der andere 49 aus seiner 46 gezogen. Entsprechende distale Drehbewegung kann durch umgekehrt kreuzend verlaufende Drähte zwischen Vorrichtungsunterarm und -hand erreicht werden.

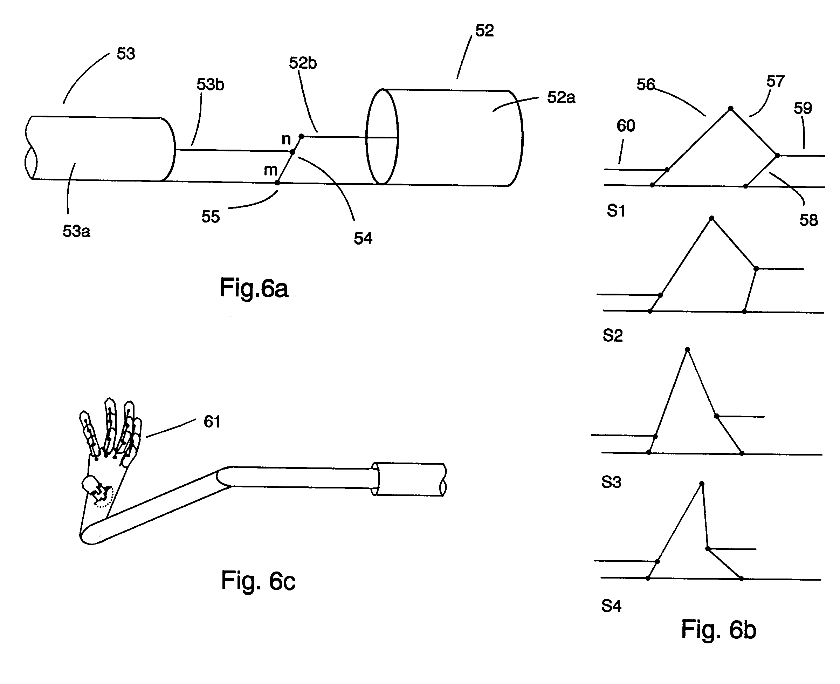

Anhand der Figur 6 soll die in Anspruch 2 dargelegte Idee erläutert werden. Dem Anspruch 2 liegt das Problem zugrunde, die verschiedenen Größenverhältnisse der menschlichen Hand des Operateurs auf die der Vorrichtungshand zu übertragen. Das Problem kann zum einen dadurch gegeben sein, daß die Vorrichtungshände andere Dimensionen aufweisen. Zum anderen kann das Problem aus dem Wunsch herrühren, spezielle Handbewegungen verändert wirken zu lassen. Ein Beispiel hierzu wäre das übermäßige Kippen des Fingerendgliedes gegen das Fingermittelglied mit einem Winkelübertragungsverhältnis von z.B. γ''/γ'=2, während die anderen Gelenke ein winkelgetreues Verhältnis von γ''/γ'=1 aufweisen. Die in Anspruch 2 dargelegte Lösung dieses Problems nutzt die Technik von Hebeln und Stangenkonstruktionen nach Art eines Pantographen.

Das Anpassen der Weglängen ist schon an dem Hebel 34''' in Fig. 2 erläutert worden. Eine andere Bauweise hierzu zeigt Fig. 6a. Zwei Bowdenzüge 52 und 53 sind mit ihren Hüllen 52a und 53a verbunden. Die Drähte 52b und 53b sind drehbar und - anders als bei Hebel 34''' der Fig. 2 - gleichseitig zum Drehpunkt 55 an der Koppelstange 54 angebracht, so daß hier keine Wegrichtungsänderung auftritt. Wird nun Draht 52b aus seiner Hülle 52a heraus-, so wird Draht 53b in seine Hülle 53a hineingeschoben. Das Verhältnis der Wegänderung des Drahtes 52b zu Draht 53b ergibt sich z.B. aus dem Strahlensatz unter Kenntnis der variabel einstellbaren Koppelstangenabgriffe n und m.

Fig. 6b zeigt das Wesen des pantographischen Mechanismus in deutlicherer Form. Es handelt sich um ein Übertragungsglied mit drei Koppelstangen 56, 57 und 58 und nichtkonstanter Winkelübersetzung (γ''/γ' ≠ konstant, siehe Teilbilder I und II der Fig. 2). Dies ist exemplarisch an vier Bewegungsstadien s1, s2, s3 und s4 der Bewegung verdeutlicht. In den Stadien s1 und s2 wird durch konstantes Voranschieben des Drahtes 59 auch der Draht 60 vorangeschoben. Ab Stadium s3 dreht sich jedoch dann die Bewegungsrichtung des Drahtes 60 um, obwohl Draht 59 weiter konstant vorangeschoben wird, Stadium 4. Es ist daher möglich, für spezielle Operationen gezielte Bewegungsabläufe zu steuern, die für die menschliche Hand nicht erreichbar wären. Hierfür ist in Fig. 6c beispielsweise auch das Umklappen der Vorrichtungshand 61 um einen für die Menschenhand unnormalen Winkel gezeigt.

Durch solche Übersetzungsglieder sowohl durch die Wahl der in Fig. 2 gezeigten Abstände a' und b' der proximalen sowie a'' und b'' der distalen Glieder ist es möglich, aus einem vielseitigen einen speziell einsetzbaren Manipulator zu machen.

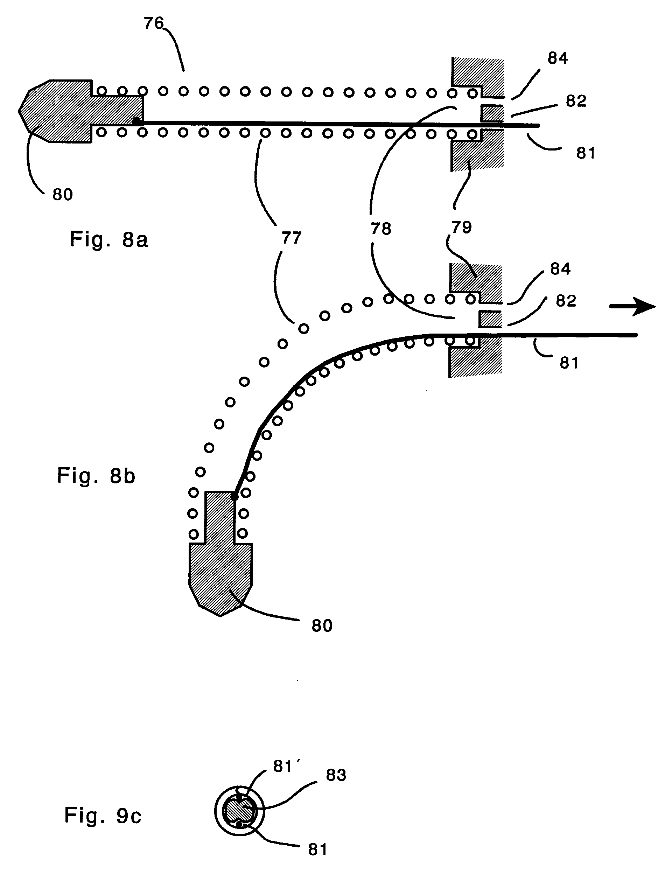

Anspruch 3 soll anhand des Folgenden sowie an Figur 7 erklärt werden. In der Medizintechnik müssen Geräte meist so beschaffen sein, daß sie sterilisierbar sind. Materialien halten z.B. in den zu diesem Zweck genutzten Heißdampföfen (Autoklaven) die Temperaturen oder die Luftfeuchtigkeit nicht aus oder es müssen Geräte auseinandergebaut werden, damit auch alle verborgenen Hohlräume gereinigt und anschließend sterilisiert werden können. Dies Problem läßt sich für die hier vorgestellte Vorrichtungshand dadurch umgehen, daß dieser Hand, ähnlich der menschlichen Chirurgenhand, ein gummielastischer Handschuh übergezogen wird. Dieser kann dann nach der Operation weggeworfen werden. Die Vorrichtungshand selbst braucht dann nur noch desinfiziert zu werden.

Für besonders kleine distale Extremitäten kann ein gummielastischer Überzug auch als Gelenk dienen. Fig. 7a und -b zeigt dies an zwei Gliedern 62 und 63. Diese sind mit einem an ihrer Unterseite aufgeklebten, etwas stärkeren Gummistück 64 verbunden. Der besagte gummielastische Überzug 65 umhüllt beide Glieder 62 und 63 und andere. Beim Vorausschieben des Drahtes 66 weicht Glied 63 dem erzeugten Druck dadurch aus, daß es sich, an dem aufgeklebten Gummistück 64 haltend, gegen Glied 62 beugt, Fig. 7b. Die dabei gespannte Gummihülle kann dann durch Entspannen das Glied 63 wieder in seine Ausgangslage zurückziehen, Fig. 7a.

Anhand der Figur 8 soll Anspruch 4 erläutert werden. Bei den in Fig. 1 und 5 gezeigten freilaufenden Drähten 18, 49 und 50 kann ein seitliches Ausscheren der Drähte und somit ein Übertragungsfehler auftreten. In Fig. 8 ist nun ein menschlicher Finger 4° gezeigt, dessen Bewegung des Fingermittengliedes 67 gegen das Fingergrundglied 68 mittels eines Gelenkes 69 und eines auf dem Finger liegenden Bowdenzuges 71 vorgenommen wird. Der Draht 70 ist im freilaufenden Teil mit einer Spiralfeder 74 ummantelt, die den Draht ohne auszuscheren sicher in dessen Hülle 75 leitet.

Das Prinzip einer neuartigen Manipulatorhand ist im Querschnitt in Fig. 8 anhand eines Fingers 76 gezeigt. Der Finger 76 besteht aus einer Spiralfeder 77, die in eine Bohrung 78 am Handgrundteil 79 eingelassen ist. Die Spiralfeder 77 ist mit einem Metallbolzen 80 am Fingerende verbunden. Ein Seil 81 ist mit dem Metallbolzen 80 fest verbunden und verläuft innerhalb und entlang der Feder 77 durch eine Bohrung 82 des Handteils 79. In Fig. 8a liegt das Seil 81 locker, in Fig. 8b wird es von dem proximalen Ende gezogen, siehe Pfeil. Aufgrund des Ziehens wird die Spiralfeder gekrümmt, der Finger biegt sich.

Innerhalb der Spiralfeder 77 kann sich zur besseren Führung des Seiles 81 ein Gummiprofil 83 befinden, welches in Fig. 8c gezeigt ist. Über weitere Bohrungen 84, Fig. 8a und b, können weiter Seile, z.B. 81', zur Erweiterung der Krümmungsmöglichkeiten des Fingers eingeführt werden.

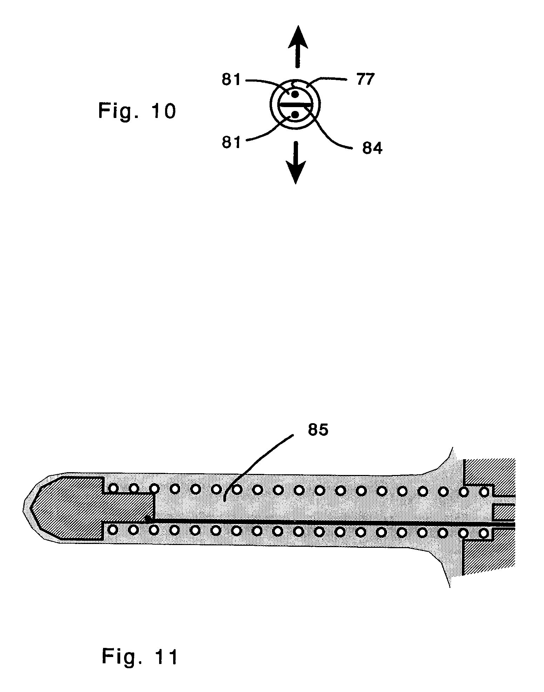

In Fig. 10 ist ein Finger im Querschnitt gezeigt, dem durch ein in die Spiralfeder 77 eingebrachtes dünnes Blech 84 Stabilität und eine Vorzugskrümmung verliehen wurde. Er krümmt sich vorzugsweise in die mit Pfeilen angedeuteten Richtungen.

Zur besseren Reinigung bzw. Sterilisation ist anhand eines Fingers in Fig. 11 der Einguß in ein Gummi 85 gezeigt. Hier entstehen keine schwer zu reinigenden Hohl- und Zwischenräume, das Innenprofil 83 wird durch diese Bauart ersetzt und der Finger kann sich mit seiner Spirale während des Arbeitens mit dem Manipulator nicht an fremden Gegenständen festhaken. Obendrein kann der distalen Hand somit ein Aussehen verliehen werden, welches einer menschlichen ähnlich sieht, so daß dem Operateur durch diese Suggestion ein für das Gelingen seiner Arbeit wichtiges und bekanntes Verhältnis zur Umwelt gegeben wird.

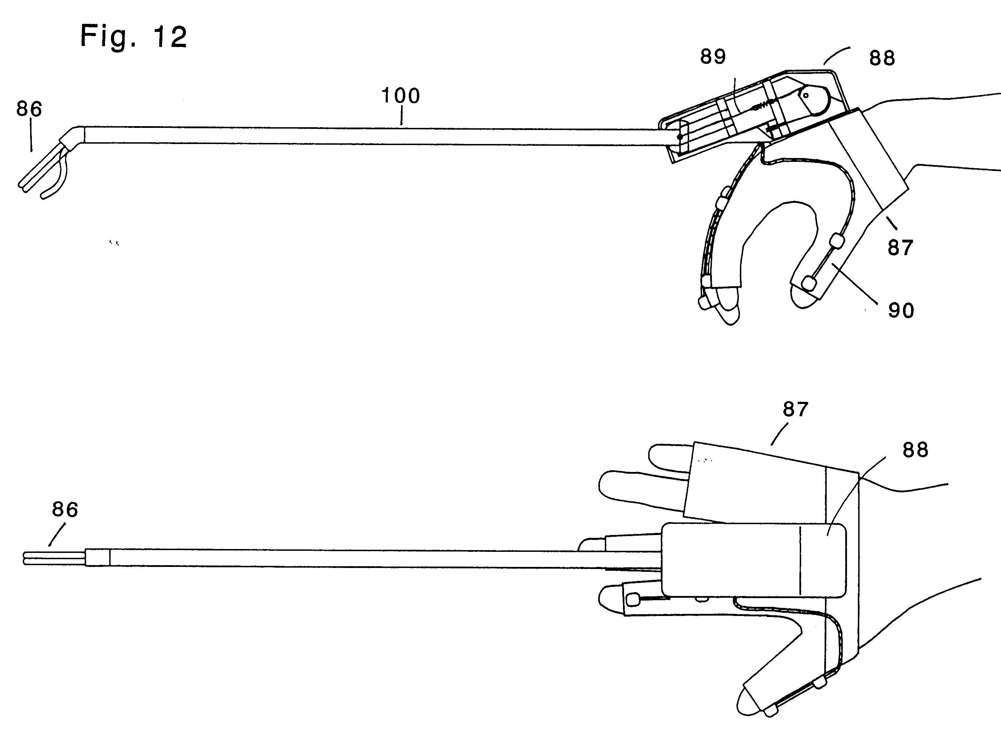

Es sollen nun weitere Möglichkeiten für den Sitz des Koppelgliedes erläutert werden. In Fig. 12 ist in Seitensicht und Draufsicht ein Manipulator gezeigt, dessen Koppelglied 88 auf der Oberfläche des proximalen Teils 97 befestigt ist. Dieser Teil der menschlichen Hand bewegt sich kaum und bietet daher einen guten Halt für das Koppelglied 88. Die Bouwdenzüge 90 verlaufen auf dem proximalen Handschuh, wie gezeigt, oder können in das elastische Material des Handschuhs eingearbeitet sein. Im Koppelglied 88 verlaufen die Inntenteile der Bouwdenzüge 89 wieder frei, ohne Hülle. Der Bowdenzug der Daumenteile können auch in der Hohlhand verlaufen.

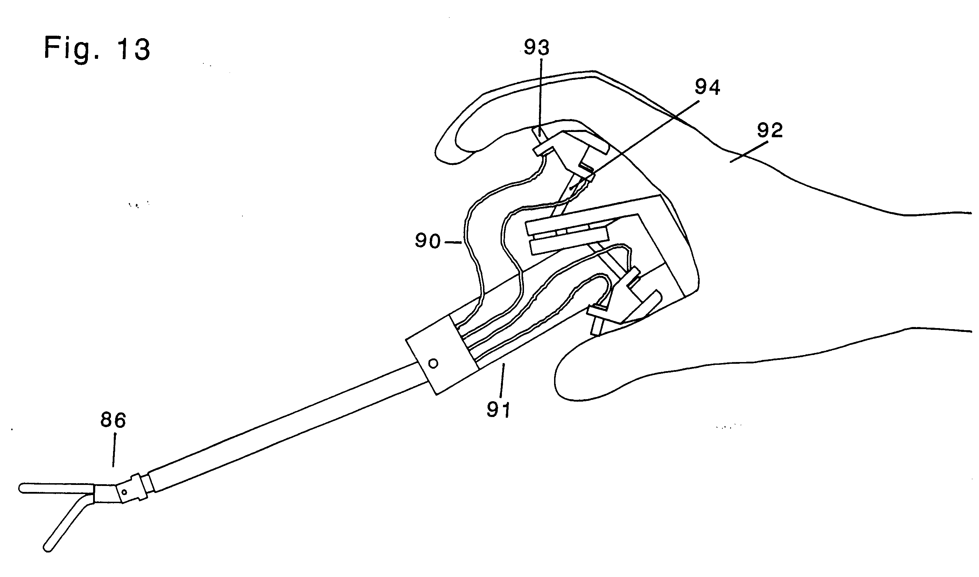

Fig. 13 zeigt einen Manipulator, bei dem das Koppelglied 91 im Hohlraum des proximalen Teils liegt. Die Menschenhand 92 umgreift den Manipulator. Hier liegt der Vorteil in der festeren und stabileren Bauweise des Manipulators. Denn die die Bewegung der Finger aufnehmenden Stössel 93 und 94 können massiver gebaut und daher stärker belastet werden. Mit einem so gearteter Manipulator kann man fester zugreifen.

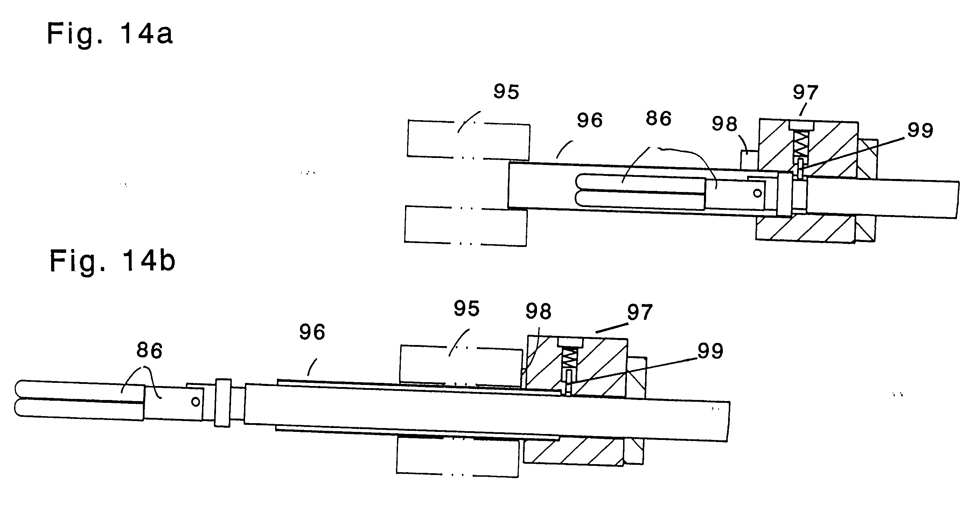

Fig. 14 zeigt eine Schutzvorrichtung für die distale Manipulatorhand 86. Bei den filigranen distalen Fingerstrukturen kann es dazu kommen, daß diese beim abrupten einziehen des Manipulators in die Trokarhülse beschädigt werden. Eine Schnappvorrichtung 97 mit einer Schutzhülle 96 verhindert dies. In Fig. 14a liegt die distale Hand 86 ganz in ihrer Schutzhülle 96. Dies entspricht dem Einschieben des Manipulators in die Trokarhülse. Wird dann das Gerät mit dem Druckschalter 98 auf den Trokar 95 gedrückt, wird ein Sperrbolzen 99 derart hochgezogen, daß der Manipulator sich von seiner Schutzhülle 96 lößt und frei an den Ort der Operation geschoben werden kann, Fig 14.b. Der Mechanismus funktionier auch in umgekehrter Richtung.

Eine weitere Ausgestaltung besteht darin, daß die distale Hand um die Transferstange 100 gedreht werden kann. Man erhält somit eine weitere Bewegungsfreiheit. Hierzu könnte sich auf der Transferstange 100 ein Griff für die zweite Hand des Operateurs befinden, mit der diese Rotationsbewegung durchgeführt werden kann.

## Patentansprüche

1. Mechanische Manipulatorvorrichtung zum Arbeiten in unzugänglichen Gebieten und Hohlräumen,
gekennzeichnet durch
den proximalen Teil,
der eine paßgenaue Halterung für eine menschliche, sich aus der Hand mit Fingern, Fingergliedern und Körper, dem Untersowie dem Oberarm oder nur Teilen dieser sich ergebenden Extremität darstellt, und aus Gliedern besteht, die direkt an denen der menschlichen Extremität ansetzen und die Bewegungen dieser mitmachen,
den distalen Teil,
der seiner Form und Funktion nach wie eine menschliche Extremität wirkt, aus beweglich miteinander verbundenen Gliedern besteht, die je ein Gegenstück eines Gliedes des proximalen Teils darstellen und zusammengelegt durch eine enge Öffnung geschoben werden kann, und
dem Übertragungsteil der Bewegung,
der aus Drähten bzw. Fäden - Bowdenzügen - besteht, die je ein Glied des proximalen mit einem des distalen Teils verbinden und an diesen Gliedern beweglich befestigt sind, so daß die von einem menschlichen Glied ausgeführte Bewegung direkt auf das entsprechende Glied des distalen Teils durch Schub oder Zug übertragen wird.

2. Vorrichtung nach Anspruch 1,
dadurch gekennzeichnet,
daß die Bewegung der Drähte und Fäden mittels Hebel oder pantographisch arbeitenden Einrichtungen in ihrer Wegrichtung und -länge verändert werden können.

3. Vorrichtung nach Anspruch 1 und 2,
dadurch gekennzeichnet,
daß die am distalen Ende befindliche Vorrichtungsextremität einen gummielastischen Überzug bzw. eine auswechselbare gummielastische Hülle tragen und daß dieser Überzug bzw. diese Hülle auch als Gelenk dienen kann.

4. Vorrichtung nach Anspruch 1 bis 3,
dadurch gekennzeichnet,
daß das Einfahren der Drähte in die Hüllen der Bowdenzüge durch Spiralfedern unterstützt werden kann.

5. Vorrichtung nach Anspruch 1 bis 4
dadurch gekennzeichent, daß
die Glieder der Hand und des ansetzenden Armes aus Spiralfedern aufgebaut sind, die mit in sich verlaufenden Seilen, Fäden oder Drähten verbunden sind, so daß sich die Spiralfedern durch mechanischen Zug an den proximalen Enden dieser Seile, Fäden, Litzen oder Drähte krümmen lassen und so die Glieder der Hand und des Arms bewegt werden können.

6. Vorrichtung nach Anspruch 1 bis 5
dadurch gekennzeichnet, daß
dünne Bleche, die innerhalb und entlang der Spiralfedern angebracht sind, die Krümmung der Spiralfedern in einer gewünschten Richtung unterstützen.

7. Vorrichtung nach Anspruch 1 bis 6
dadurch gekennzeichent, daß
dieser gesamte distale Manipulatorteil in Gummi eingegossen ist.
